# EUROPEAN PATENT SPECIFICATION

(11) **EP 1 283 725 B1**
(45) Date of publication and mention of the grant of the patent: **19.07.2006**
(21) Application number: 01934563.6
(22) Date of filing: 17.05.2001
(51) Int. Cl.: A61K 47/30, A61K 31/41, A61K 9/14

(54) **SOLID DISPERSION SYSTEM OF PRANLUKAST WITH IMPROVED DISSOLUTION, AND THE PREPARING METHOD THEREOF**
FESTES DISPERSIONSSYSTEM VON PRANLUKAST MIT VERBESSERTER AUFLÖSUNG UND METHODE ZU DESSEN HERSTELLUNG
SYSTEME DE DISPERSION SOLIDE DE PRANLUKAST AVEC DISSOLUTION AMELIOREE ET TECHNIQUE DE PREPARATION DE CELUI-CI

(30) Priority: 20.05.2000 KR 2000027237
(43) Date of publication of application: 19.02.2003
(73) Proprietor: PEGSPHERE Co., Ltd., Suwon 440-746 (KR)
(72) Inventor: Lee, Sang Deuk, Seoul 135-280 (KR)
(74) Representative: Barz, Peter
(86) International application number: PCT/KR2001/000804
(87) International publication number: WO 2001/089574

(56) References cited:
- EP-A- 0 834 314
- WO-A-96/41628
- OTSUKA M ET AL: "Hygroscopic stability and dissolution properties of spray-dried solid dispersions of furosemide with Eudragit." JOURNAL OF PHARMACEUTICAL SCIENCES. UNITED STATES JAN 1993, vol. 82, no. 1, January 1993 (1993-01), pages 32-38, XP009009772 ISSN: 0022-3549
- SCHEEN P-C ET AL: "FORMULATION STUDIES OF A POORLY WATER-SOLUBLE DRUG IN SOLID DISPERSIONS TO IMPROVE BIOAVAILABILITY" INTERNATIONAL JOURNAL OF PHARMACEUTICS, AMSTERDAM, NL, vol. 118, no. 2, 1995, pages 221-227, XP001030819 ISSN: 0378-5173
- KAWASHIMA ET AL.: 'A new powder design method to improve inhalation efficiency of pranlukast hydrate dry powder aerosols by surface modification with hydroxypropylmethyl cellulose phthalate nanospheres' PHARM. RES. vol. 15, no. 11, 1998, pages 1748 - 1752, XP002965705

## Description

### Technical Field

The present invention is related to a novel composition of pranlukast whose solubility in aqueous media is low, a method for preparing said composition and oral preparation including the novel composition.

### Background Art

4-oxo-8-[4-(4-phenylbutoxy)benzoyl-amino]-2-(tetrazol-5-yl)-4H-1-benzopyran hemihydrate (general name: pranlukast; hereinafter, referred to as pranlulkast in the present specification including the patent claims) is a strong antagonist against leukotriene C4(LTC4) and leukotriene D4(LTD4) and used as therapeutic agent for bronchial asthma and allergic rhinitis, and is expected as a therapeutic agent for allergic shock and various allergic inflammation, very broad in application range.

However, pranlukast is very slightly soluble in water and its bioavailability is low upon oral administration, thus actual application to patients needs administration of a large amount of drug, leading to economic loss. Therefore, development of preparation with improved oral absorption was urgently required, but no preparation with improved oral absorption through improving dissolution of pranlukast has been developed.

As prior reference for pharmaceutical composition of pranlukast, there are International Patent Laid-open No. WO96/41628 concerning a granules containing pranlukast, manufacturing method thereof and a method for lowering cohesiveness of pranlukast, Japanese Patent Laid-open Pyung8-73353 as to a preparation comprising pranlukast and polyvinylpyrrolidone or β -cyclodextrin, International Patent Laid-open No. WO99/04790 on aqueous pharmaceutical composition containing as a major component benzopyran derivative, and studies on pranlukast powder aerosol with improved inhalation efficiency through surface modification (Pharmaceutical Research 1998, 15, 1748-1752), however these disclosures completely differ from the present invention for solid dispersion composition by which oral absorption can be increased due to improved dissolution of pranlukast for the reasons described below.

First, said International Patent Laid-open WO96/41628 prepared the granules by dissolving sugars, water-soluble polymer and/or surfactant into purified water to lower the cohesiveness of pranlukast, by suspending pranlukast and by spray drying, thereby allowing the formulation process to be easier by improvement of the surface properties of pranlukast. As the International Patent Laid-open WO96/41628 suspended pranlukast powder into aqueous solution where additives are dissolved and dried by spray drying, as can be confirmed by powder X-ray diffraction analysis, crystallinity of pranlukast is retained as it is, thus dissolution of pranlukast is not greatly improved. Filling for commercial Onon® capsule prepared by International Patent Laid-open WO96/41628 exhibits very low dissolution rate, less than 5%, at 37 °C and pH 6.8.(The commercial preparation Onon® capsule described in Experimental Examples 5 and 6 of the present specification is the product by this prior art.)

Japanese Patent Laid-open Pyung 8-73353 relates to liquid preparations such as eye drop, nasal drop or injections using as a solubilizer polyvinylpyrrolidone or β - cyclodextrin, and International Patent Laid-open WO99/04790 relates to liquid preparation such as pranlukast aqueous solution including surfactant and pranlukast suspension including water-soluble polymer. These preparations may be orally administered as solution or suspension, yet in case of solution, for administering usual dose, several hundred milliliters(mℓ) per once should be administered due to too low concentration of pranlukast, thus inadequate for oral preparation, further when solubility is increased through control of pH of the solution, precipitation due to gastric acid might be occurred upon oral administration. In addition, in case of suspension, as crystallinity of pranlukast is retained, it is difficult to expect substantial improvement in dissolution or oral absorption rate of pranlukast.

It is known that solid dispersion which is used as a method for improving oral absorption of slightly soluble drug increases the oral absorption by improving in vitro and in vivo dissolution property of the drug. Solid dispersion is a type of mixture where at least one active component is uniformly dispersed in polymer of solid state or inert carrier and can be prepared by method such as coprecipitation, coevaporation, freeze drying, spray drying and cogrinding etc. (*J. Pharm. Sci.* 1993, 82, 32-38). As an example for such a case where dissolution rate of drug is improved through preparation of solid dispersion of the drug, dicumarol-polyethyleneglycol solid dispersion (*J. Pharm. Sci.* 1981, 70, 1353-7), mefenamic acid-polyethyleneglycol solid dispersion (*Pharm. Develop. Technol.* 1998, 3, 405-412), beads having a core coated with an antifungal and a polymer (International Patent Laid-open WO94/05263), diazepam-polyethyleneglycol solid dispersion (*Pharm. Acta. Helv.* 1989, 64, 90-93), furosemide-polyvinylpyrrolidone solid dispersion (*J. Pharm. Pharmacol.* 1989, 41, 73-78), nifedipine-enteric coating agent solid dispersion (*Chem. Pharm. Bull.* 1985, 33, 388-391), enteric solid dispersion of cyclosporin (*Chem. Pharm. Bull.* 1989, 37, 2542-2544), tolbutamide-enteric coating polymers solid dispersion (*Chem. Pharm. Bull.* 1987, 35, 3800-3806) and triazole-based antifungal agent solid dispersion (*Chem. Pharm. Bull.* 1996, 44, 568-571) can be enumerated.

However, it is not always true that conversion of slightly soluble drug into solid dispersion by using carrier achieves enough dissolution rate for oral absorption. Even in the above examples, almost no one raised drug dissolution above 85%. For example, the dissolution rate of the solid dispersion of triazole-based antifungal agent MFB-1041 containing hydroxypropylcellulose, hydroxypropylmethylcellulose, hydroxypropylmethylcellulose phthalate and carboxymethylethylcellulose was reported to be less than 12% and the dissolution rate after 1 hour, only 6%.

In case of pranlukast, there was no report on improvement of dissolution properties and oral absorption rate via preparation of solid dispersion, nor any study as to solid dispersion of the same series, leukotriene antagonist was reported.

The inventor of the present invention selected solid dispersion as a method for improving the low bioavailability of slightly soluble pranlukast, and conducted intensive studies to complete the present invention.

The objective of the present invention lies in providing a composition and method for preparation thereof, which exhibits far increased bioavailability due to improvement of in vitro and in vivo dissolution properties of pranlukast and which is suitable for the manufacture of oral preparation.

### Disclosure of the invention

The present invention relates to a novel composition of pranlukast, of which solubility in aqueous media is low, method for producing said composition and oral preparation comprising said composition. More specifically, the present invention is related to oral solid dispersion composition of pranlukast with improved dissolution characterized in comprising (A) pranlukast and (B) at least one polymer selected from a group consisting of hydroxypropylcellulose and hydroxypropylmethylcellulose; method for preparation thereof; and oral preparation prepared thereby.

To raise absorption rate of slightly soluble drug by improving dissolution via preparation of solid dispersion thereof, it is important to determine the kind of carrier and composition ratio by which dissolution in the intestinal tract can be retained at a high level. The composition of the present invention was designed to enable a great deal of dissolution to occur in the intestinal tract, absorption site of pranlukast.

As result of intensive studies on various polymers, it was found out that such purpose could be accomplished by use of the said polymer.

The weight ratio of polymer used to drug is preferably 0.25:1 to 5:1, more preferably 0.5:1 to 3:1. Too small ratio reduces dissolution, whereas increased ratio causes difficulty in manufacturing preparation containing the solid dispersion composition. That is, to manufacture said solid dispersion composition into tablets, capsules, granules or dry syrup, granulation such as dry granulation is necessary, and if the amount of polymer contained in solid dispersion is too much, compression molding is difficult due to cohesiveness of polymer and disintegration of prepared granules or tablets is retarded to lead to retarded dissolution, resulting in necessity for use of an excessive amount of diluents.

In addition, the composition of the present invention can further comprise hydroxypropylmethylcellulose phthalate 50. At this time, though the amount of use can substitute a part of the total amount of the polymer, when the weight ratio of hydroxypropylmethylcellulose phthalate 50 to said polymer exceeds 4:1, dissolution of pranlukast is reduced, thus the ratio is preferably 0.1:1 to 4:1, more preferably, 0.25:1 to 2:1.

When comparing with pranlukast solid dispersion without addition of hydroxypropylmethylcellulose phthalate 50, the pranlukast solid dispersion prepared by substituting a part of hydroxypropylcellulose or hydroxypropylmethylcellulose contained in solid dispersion composition with hydroxypropylmetixylcellulose phthalate 50 as described above, exhibits faster disintegration while showing equal dissolution at 37°C and pH 6.8.

The pranlukast solid dispersion composition according to the present invention is very remarkable for the improved dissolution, at least 85%, even up to almost 100%, unlike conventional solid dispersion composition of various drugs and this effect is directly reflected in real bioavailability.

Another embodiment of the present invention, method for preparing the solid dispersion composition of the present invention comprises dissolving pranlukast and the polymer(s) in a mixture of dichloromethane-methanol and drying the solvent. As the volume ratio between dichloromethane and methanol in the mixed solvent, 2:1 to 5:1 is preferable, and 3:1 to 4.5:1 is more preferable. Solvent can be dried by drying method such as spray drying, vacuum drying and etc. using spray drier, fluidized bed granulator, CF granulator, vacuum drier and etc.

In order for effective production of solid dispersion of pranlukast, it is essential to melt pranlukast and carrier or to dissolve them in a solvent. However, in case of pranlukast, the melting temperature and decomposition temperature are very close, so safe melting is difficult. Furthermore, pranlukast is very low soluble in organic solvents. Therefore, preparation of solid dispersion is not easy. The inventor of the present invention found as result of intensive studies on various organic solvents that pranlukast can be easily dissolved to a concentration of 1% or more by adding pranlukast into dichloromethane-methanol mixture and by heating, if necessary. Said solvent composition easily dissolves the polymer used in the present invention, thus is adequate for preparing solid dispersion of pranlukast and polymer carrier. For the mixture, though ethanol can be used instead of methanol, it is more difficult to obtain concentrated solution compared to methanol. In case of using single solvent, methanol or dichloromethane or in case of using other alcohol or haloalkane, it is difficult to prepare at least 1% solution necessary for effective mass production of pranlukast solid dispersion.

Additionally, pranluakst solid dispersion composition of the present invention can further comprise surfactant, preservatives, complex-forming agent, electrolyte, discharging agent or other active ingredient, e.g. various components that can be used in combination with pranlukast such as steroid, bronchodilator or antitussive expectorant.

The solid dispersion composition of pranlukast of the present invention can be formulated into pharmaceutical dosage forms containing therapeutically effective amount of medicinal compound. Though oral dosage form such as tablet, capsule, granule and dry syrup is primarily considered, the composition of the present invention can be used for the preparation of pharmaceutical dosage form such as eye drop, nasal spray or inhalation. Preferred form is oral dosage form formulated into tablets. These can be manufactured by using conventional component or diluents, and conventional tablet machine and tablet-preparing technique. Tablets can include one or more additives, for example, diluents, disintegrator, binders, lubricants, gliding agent, sweetening agent, flavoring agent and coloring agent. Some of them may be used for more than one purposes. Tablets of the present invention can be film-coated for the purpose of improvement in stability, flavor, convenience of intake and appearance.

### Brief Description of Drawings

Fig. 1 illustrates dissolution rate of pranlukast solid dispersion composition (Comparative Examples 1~8, and Examples 5 and 7) containing polymer in a weight ratio against drug, 1.5:1, depending on the kinds of polymer at 37°C and pH 6.8.
Fig. 2 illustrates dissolution rate of pranlukast solid dispersion composition (Examples 1~6) containing hydroxypropylcellulose depending on the weight ratio of hydroxypropylcellulose against drug at 37 °C and pH 6.8.
Fig. 3 illustrates dissolution rate of pranlukast solid dispersion composition (Examples 8~12) containing hydroxypropylcellulose and hydroxypropylmehylcellulose phthalate 50 (polymer: drug, 1:1 by weight) depending on the weight ratio of hydroxypropylmethylcellulose phthalate 50 against hydroxypropylcellulose at 37°C and pH 6.8.
Fig. 4 illustrates dissolution rate of pranlukast solid dispersion composition (Examples 17, 16, 10, 15, 14 and 13) containing polymer of hydroxypropylcellulose: hydroxypropylmehylcellulose phthalate 50 (1:1) depending on the weight ratio of polymer to drug at 37 °C and pH 6.8.
Fig. 5 illustrates dissolution rate of 1 tablet of Example 18 (pranlukast 120 mg), commercial preparation, two Onon® capsules (pranlukast 225 mg) and pranlukast bulk powder (225 mg) at 37 °C and pH 6.8.
Fig. 6 illustrates plasma concentration of pranlukast (n=6) upon separate administration to adult healthy male of one of the tablet of Example 18 (pranlukast 120 mg) and two Onon® capsules (pranlukast 225 mg).
Fig. 7 illustrates powder X-ray diffraction test result for pranlukast crude powder (A), conventional preparation Onon® capsule filling (B), hydroxypropylcellulose (C), hydroxypropylmethylcellulose(D), hydroxypropylmethylcellulose phthalate 50 (E), talc (F), Example 4 (G), Example 7 (H) and Example 10 (I).

### Examples

### Example 1. Preparation of solid dispersion composition

Solid dispersion composition was prepared according to the composition described in Table 1. Pranlukast 10 g and as polymer, hydroxypropylcellulose 1.25 g were mixed and methanol 200 ml and dichloromethane 800 ml were added and with stirring, dissolved by heating to 40°C. To the mixture, talc 0.5 g was added and dried under stirring in spray drier by spraying to a speed of 70 ml/min. At this time, inlet air temperature was 95~105°C, outlet air temperature was 40~50°C, and jet pressure was controlled to be about 3 kg/cm².

**Table 1. Composition of Examples 1~7**

| Examples | Pranlukast (g) | Polymer (g) | | Talc (g) |
|---|---|---|---|---|
| | | Hydroxypropyl cellulose | Hydroxypropyl methylcellulose | |
| 1 | 10 | 1.25 | 0 | 0.5 |
| 2 | 10 | 2.5 | 0 | 0.5 |
| 3 | 10 | 5 | 0 | 0.5 |
| 4 | 10 | 10 | 0 | 0.5 |
| 5 | 10 | 15 | 0 | 0.5 |
| 6 | 10 | 30 | 0 | 0.5 |
| 7 | 10 | 0 | 15 | 0.5 |

### Examples 2 to 7. Preparation of solid dispersion composition

According to the composition as listed in Table 1, solid dispersion compositions were prepared separately. The manufacturing method was the same as in Example 1.

### Comparative Examples 1 to 8. Preparation of solid dispersion composition

According to the composition as represented by Table 2, solid dispersion compositions were prepared separately and the method was the same as in Example 1.

**Table 2. Composition for Comparative Examples 1~8**

| Comparative Example | Pranlukast (g) | Polymer (g) | | Talc (g) |
|---|---|---|---|---|
| 1 | 10 | polyvinylpyrrolidone | 15 | 0.5 |
| 2 | 10 | polyethyleneglycol 6000 | 15 | 0.5 |
| 3 | 10 | hydroxypropylmethylcellulose phthalate50 | 15 | 0.5 |
| 4 | 10 | hydroxypropylmethylcellulose phthalate 55 | 15 | 0.5 |
| 5 | 10 | Ethylcellulose | 15 | 0.5 |
| 6 | 10 | Poloxamer | 15 | 0.5 |
| 7 | 10 | Eudragit E | 15 | 0.5 |
| 8 | 10 | Eudragit L | 15 | 0.5 |

### Examples 8 to 12. Preparation of solid dispersion composition

According to the composition shown in Table 3, solid dispersion compositions were prepared separately. The polymer ratio to drug was fixed at 1:1 and only the weight ratio between the two polymers was changed. The preparation method was the same as in Example 1.

**Table 3. Composition for Examples 8 to 12**

| Example | Pranlukast (g) | Polymer (g) | | Talc (g) |
|---|---|---|---|---|
| | | Hydroxypropyl Cellulose | Hydroxypropylmethylcellulose phthalate 50 | |
| 8 | 10 | 8.00 | 2.00 | 0.5 |
| 9 | 10 | 6.67 | 3.33 | 0.5 |
| 10 | 10 | 5.00 | 5.00 | 0.5 |
| 11 | 10 | 3.33 | 6.67 | 0.5 |
| 12 | 10 | 2.00 | 8.00 | 0.5 |

### Examples 13 to 17.

According to the composition given in Table 4, solid dispersion compositions were prepared separately. The weight ratio of the two polymers was fixed at 1:1 and only the weight ratio of polymer against drug was changed. The method was the same as in Example 1.

**Table 4. Composition for Examples 13 to 17**

| Example | Pranlukast (g) | Polymer (g) | | Talc (g) |
|---|---|---|---|---|
| | | Hydroxypropylcellulose | Hydroxypropylmethylcellulose phthalate50 | |
| 13 | 10 | 20.00 | 20.00 | 0.5 |
| 14 | 10 | 10.00 | 10.00 | 0.5 |
| 15 | 10 | 7.50 | 7.50 | 0.5 |
| 16 | 10 | 2.50 | 2.50 | 0.5 |
| 17 | 10 | 1.25 | 1.25 | 0.5 |

### Example 18. Preparation of tablets using solid dispersion composition

According to conventional method for preparing tablets, tablets containing pranlukast 120 mg was prepared from the solid dispersion composition prepared in Example 10. Actual tablet composition is as listed in Table 5.

**Table 5. Tablet Composition for Example 18**

| | Component | Content (mg)/tablet | Content (%)/tablet |
|---|---|---|---|
| Tablet core | pranlukast | 120 | 20.00 |
| | hydroxypropylcellulose | 60 | 10.00 |
| | hydoxypropylmethylcellulose phthalate50 | 60 | 10.00 |
| | talc | 6 | 1.00 |
| | lactose | 175 | 29.17 |
| | sodium chloride | 30 | 5.00 |
| | premelose | 63 | 10.50 |
| | Compritol | 48 | 8.00 |
| | aerosil | 5 | 0.83 |
| | Crospovidone | 18 | 3.00 |
| Sub-total | | 585 | 97.50 |
| Film Coating | hydroxypropylmethylcellulose | 13 | 2.17 |
| | propyleneglycol | 2 | 0.33 |
| Sub-total | | 15 | 2.50 |
| Total | | 600 | 100.00 |

### Experimental Example 1. Dissolution test I: Kinds of polymer

Dissolution profiles of the pranlukast solid dispersion compositions of Comparative Examples 1 to 8, Examples 5 and 7 were compared. The dissolution test was conducted according to the Method II of dissolution test (Paddle method) in U.S. Pharmacopoeia. 900 ml of second fluid (pH 6.8) was added into dissolution test device and sample corresponding to pranlukast 120 mg was added while maintaining the temperature at 37°C and time-dependent dissolution was determined under stirring with a speed of 100 rpm. At each time-point, aliquot 1 ml was taken into silicone-coated tube, centrifuged and the supernatant was subjected to HPLC analysis.

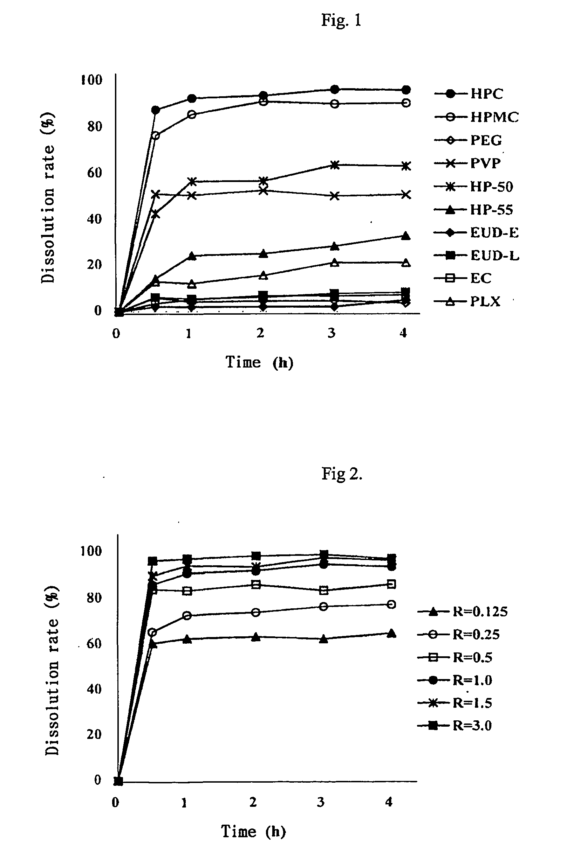

The result for dissolution test was depicted by Fig. 1. The weight ratio of polymer to drug was fixed at 1.5:1. Though the pranlukast solid dispersion of Example 5 (using hydroxypropylcellulose) and Example 7 (using hydroxypropylmethylcellulose) exhibit dissolution rate of 85% or more, pranlukast solid dispersions of Comparative Examples 1~8 using polyvinylpyrrolidone (PVP), polyethyleneglycol 6000 (PEG), hydroxypropylmethylcellulose phthalate 50 (HP-50), hydroxypropylmethylcellulose phthalate 55 (HP-55), ethylcellulose (EC), Poloxamer F-127 (PLX), Eudragit E (EUD-E) and Eudragit L (EUD-L) respectively, all exhibit dissolution rate of 65% or less.

### Experimental Example 2.

### Dissolution test II: Weight ratio of hydroxypropylcellulose against drug

Dissolution profiles of the pranlukast solid dispersion compositions of Examples 1, 2, 3, 4, 5 and 6 were compared. Test was conducted as in Experimental Example 1.

The result is illustrated in Fig. 2. The weight ratio of polymer against drug was changed, in order, 0.125:1, 0.25:1, 0.5:1, 1:1, 1.5:1 and 3:1. All solid dispersion compositions tested exhibit relatively high dissolution rate, and in particular, pranlukast solid dispersions (Examples 3~6) where the weight ratio of hydroxypropylcellulose is 0.5:1 or higher exhibit very high dissolution rate of 85% or more.

### Experimental Example 3.

### Dissolution test III: Weight ratio of hydroxypropylmethylcellulose phthalate 50 against hydroxypropylcellulose

Dissolution profiles of the pranlukast solid dispersion compositions of Examples 8, 9, 10, 11 and 12 were compared. Test was conducted as in Experimental Example 1.

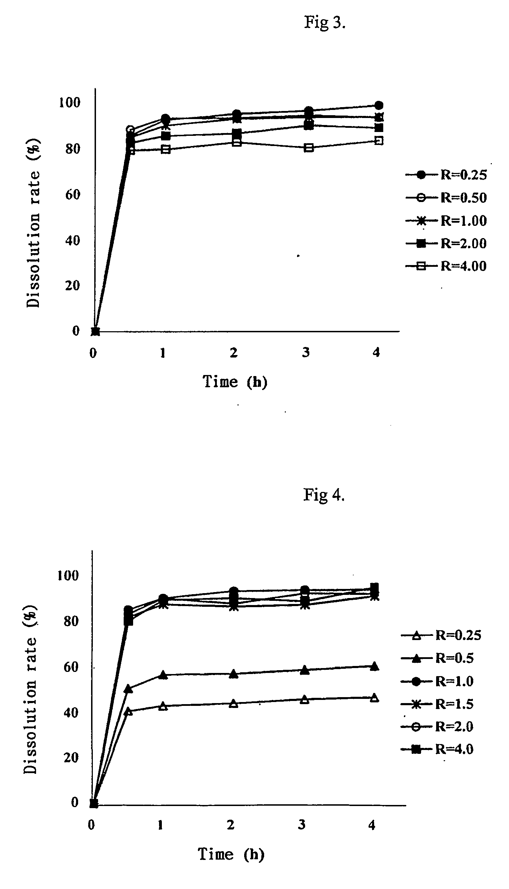

The result is shown in Fig. 3. The weight ratio of total polymer against drug was fixed at 1:1, yet the weight ratio of hydroxypropylmethylcellulose phthalate 50 against hydroxypropylcellulose was varied, in order, 0.25:1, 0.5:1, 1:1, 1.5:1, 2:1 and 4:1. Below 1:1 (Examples 8, 9 and 10), there was no significant difference in the dissolution rate, but above 2:1 (Examples 11 and 12), dissolution rate was a little reduced.

### Experimental Example 4.

### Dissolution test IV: Weight ratio of hydroxypropylmethylcellulose phthalate 50 against hydroxypropylcellulose

Dissolution profiles of the pranlukast solid dispersion compositions of Examples 17, 16, 10, 15, 14 and 13 were compared. Test was conducted as in Experimental Example 1.

The result is shown in Fig. 4. The weight ratio of hydroxypropylmethylcellulose phthalate 50 against hydroxypropylcellulose was fixed at 1:1, and the ratio of total weight of the polymer against drug was changed, in order, 0.25:1, 0.5:1, 1:1, 1.5:1, 2:1 and 4:1. Between 1:1 and 4:1 (Examples 10, 15, 14 and 13), there was no significant difference in the dissolution rate, but below 0.5:1 (Examples 16 and 17), dissolution rate was noticeably declined.

### Experimental Example 5. Comparative dissolution test

Comparative dissolution test was conducted according to the Method II of the dissolution test (Paddle method) in U.S. Pharmacopoeia for the one tablet (pranlukast 120 mg) of Example 18 prepared by using the solid dispersion of Example 10, commercial preparation Two Onon® capsules (pranlukast 112.5 mg/capsule, pranlukast 225 mg) and pranlukast bulk powder 225 mg. Other test method was identical with Experimental Example 1.

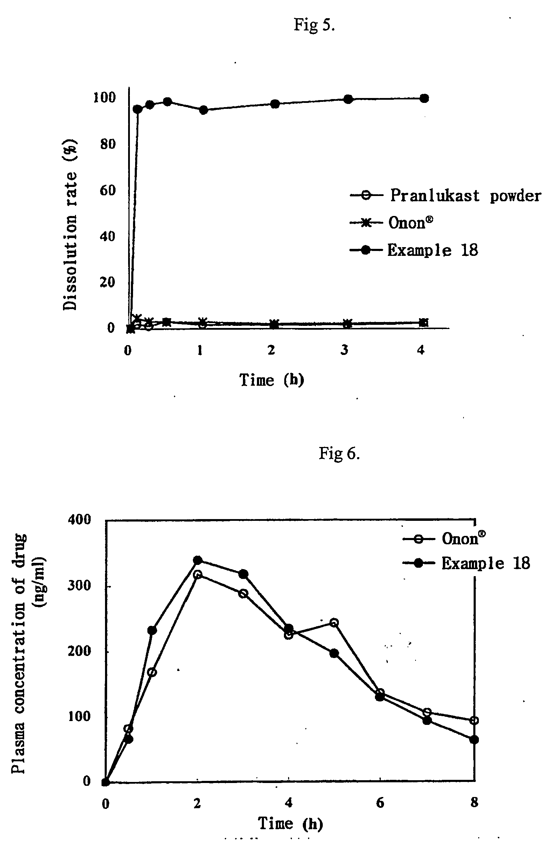

The result is as shown in Fig. 5. The dissolution rate of pranlukast bulk powder and commercial preparation was less than 5%, but that of the tablet of the present invention was 85% or more.

### Experimental Example 6. In vivo availability test

One tablet of Example 18 (pranlukast 120 mg) prepared by using solid dispersion of Example 10 and commercial preparation Two Onon® capsules (pranlukast 225 mg) were cross-administered orally to 6 healthy adult males in fasting state, and plasma concentration of pranlukast was compared.

The result is represented by Fig. 6. The plasma concentration upon administration of 1 tablet of Example 18 containing 120 mg of pranlukast was similar to that upon administration of 2 Onon® capsules, and even the area under the plasma concentration-time curve from time 0 to time 8 hour was similar, that is, 1554 ng.h/ml in test tablet and 1534 ng.h/ml in Onon® capsule.

Therefore, it could be confirmed that the tablet of Example 18 provides equal efficacy even by administering about half the amount of the conventional preparation via exhibiting a high in vivo availability.

### Experimental Example 7. X-ray Diffraction Analysis

To test crystallinity of pranlukast within the composition of the present invention, X-ray diffraction analysis was carried out. Powder X-ray diffraction analysis was conducted at room temperature by DMAX/1200 X-ray diffraction analyzer (Rikaku Denki, Tokyo, Japan) (CuKa, 40 kV, 20 mA, 5 degree/min).

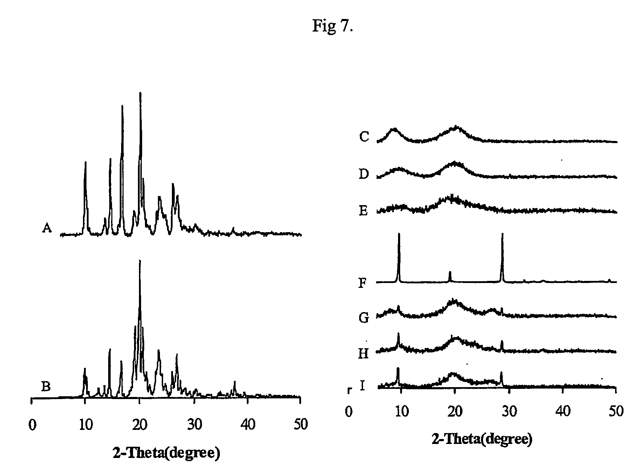

The result is illustrated in Fig. 7. In case of conventional preparation Onon® (B), crystalline peak exhibited in pranlukast bulk powder (A) was clear, confirming the crystallinity, but in case of the compositions of the present invention, Example 4 (G), Example 7 (H) and Example 10 (I), the crystalline peak existing in bulk powder was not shown. Therefore, it could be confirmed that the composition of the present invention is a solid dispersion, where pranlukast exists as amorphous form. Hydroxypropylcellulose (C), hydroxypropylmethylcellulose (D), hydroxypropylmethylcellulose phthalate 50 (E) and talc (F) are given for reference.

It was confirmed that the composition of the present invention is a solid dispersion of polymer and amorphous pranlukast that does not have crystallinity.

The composition of the present invention remarkably improves bioavailability through improving in vitro and in vivo dissolution of pranlukast, further is suitable for preparing oral dosage form, thus has economic and therapeutic benefit.

## Claims

1. A pranlukast solid dispersion composition comprising (A) pranlukast and (B) at least one polymer selected from hydroxypropylcellulose and hydroxypropylmethylcellulose.

2. The pranlukast solid dispersion composition according to claim 1, wherein the weight ratio of polymer to pranlukast is in the range of 0.25:1 to 5:1.

3. The pranlukast solid dispersion composition according to claim 1, which further comprises hydroxypropylmethylcellulose phthalate 50.

4. The pranlukast solid dispersion composition according to claim 3, wherein the weight ratio of hydroxypropylmethylcellulose phthalate 50 to at least one polymer selected from hydroxypropylcellulose and hydroxypropylmethylcellulose is in the range of 0.1:1 to 4:1.

5. A method for preparing the pranlukast solid dispersion composition according to any one of claims 1-4 **characterized by** dissolving pranlukast and the polymer(s) in a mixture of dichloromethane-methanol of a volume ratio of 2:1 to 5:1 and drying the solvent.

6. The method in claim 5, wherein the volume ratio between dichloromethane and methanol is in the range of 3:1 to 4.5:1.

## Patentansprüche

1. Feste Dispersionszusammensetzung von Pranlukast umfassend (A) Pranlukast und (B) mindestens ein Polymer ausgewählt aus Hydroxypropylcellulose und Hydroxypropylmethylcellulose.

2. Feste Dispersionszusammensetzung von Pranlukast nach Anspruch 1, wobei das Gewichtsverhältnis von Polymer zu Pranlukast im Bereich von 0,25:1 bis 5:1 liegt.

3. Feste Dispersionszusammensetzung von Pranlukast nach Anspruch 1, welche ferner Hydroxypropylmethylcellulosephthalat 50 umfasst.

4. Feste Dispersionszusammensetzung von Pranlukast nach Anspruch 3, wobei das Gewichtsverhältnis von Hydroxypropylmethylcellulosephthalat 50 zu mindestens einem Polymer ausgewählt aus Hydroxypropylcellulose und Hydroxypropylmethylcellulose im Bereich von 0,1:1 bis 4:1 liegt.

5. Verfahren zur Herstellung der festen Dispersionszusammensetzung von Pranlukast nach irgendeinem der Ansprüche 1 bis 4, **gekennzeichnet durch** das Auflösen von Pranlukast und dem oder den Polymeren in einer Mischung von Dichlormethan-Methanol mit einem Volumenverhältnis von 2:1 bis 5:1 und das Trocknen des Lösungsmittels.

6. Verfahren nach Anspruch 5, wobei das Volumenverhältnis zwischen Dichlormethan und Methanol im Bereich von 3:1 bis 4,5:1 liegt.

## Revendications

1. Composition de dispersion solide de pranlukast comprenant (A) du pranlukast et (B) au moins un polymère choisi parmi l'hydroxypropyl-cellulose et l'hydroxypropylméthylcellulose.

2. Composition de dispersion solide de pranlukast conforme à la revendication 1, dans laquelle le rapport pondéral du polymère au pranlukast est situé dans l'intervalle allant de 0,25:1 à 5:1.

3. Composition de dispersion solide de pranlukast conforme à la revendication 1, comprenant en outre du phtalate d'hydroxypropylméthyl-cellulose 50.

4. Composition de dispersion solide de pranlukast conforme à la revendication 3, dans laquelle le rapport pondéral du phtalate d'hydroxypropylméthyl-cellulose 50 au polymère au nombre d'au moins un, choisi parmi l'hydroxypropyl-cellulose et l'hydroxypropylméthylcellulose, est situé dans l'intervalle allant de 0,1:1 à 4:1.

5. Procédé de préparation d'une composition de dispersion solide de pranlukast conforme à l'une des revendications 1 à 4, **caractérisé en ce qu'**il comporte les étapes consistant à dissoudre le pranlukast et le(s) polymère(s) dans un mélange dé dichlorométhane et de méthanol en un rapport volumique de 2:1 à 5:1, puis à éliminer le solvant par séchage.

6. Procédé conforme à la revendication 5, dans lequel le rapport volumique entre le dichlorométhane et le méthanol est situé dans l'intervalle allant de 3:1 à 4,5:1.
